(19) **Europäisches Patentamt**

**European Patent Office**

**Office européen des brevets**

(11) **EP 1 260 476 B1**

(12) **EUROPÄISCHE PATENTSCHRIFT**

(45) Veröffentlichungstag und Bekanntmachung des Hinweises auf die Patenterteilung:
**22.09.2004 Patentblatt 2004/39**

(51) Int Cl.⁷: $B65H\ 63/06$, $G01N\ 33/36$

(21) Anmeldenummer: **01112448.4**

(22) Anmeldetag: **22.05.2001**

(54) **Verfahren zum Prüfen der Qualität eines Garns**

Yarn quality control method

Procédé pour contrôler la qualité d'un fil textile

(84) Benannte Vertragsstaaten:
**CH DE IT LI**

(43) Veröffentlichungstag der Anmeldung:
**27.11.2002 Patentblatt 2002/48**

(73) Patentinhaber: **Gebrüder Loepfe AG**
**CH-8620 Wetzikon (CH)**

(72) Erfinder: **Scherer, Heinrich**
**8344 Bäretswil (CH)**

(74) Vertreter: **Blum, Rudolf Emil Ernst et al**
**c/o E. Blum & Co**
**Patentanwälte**
**Vorderberg 11**
**8044 Zürich (CH)**

(56) Entgegenhaltungen:
| | |
|---|---|
| **EP-A- 0 401 600** | **EP-A- 0 423 380** |
| **CH-A- 439 796** | **CH-A- 527 761** |
| **DE-A- 19 547 544** | **US-A- 3 892 951** |
| **US-A- 5 182 457** | |

**Beschreibung**

Die Erfindung betrifft ein Verfahren zum Prüfen der Qualität eines Garns gemäss Oberbegriff von Anspruch 1.

Bei der Verarbeitung wird die Qualität des Garns regelmässig geprüft. Dies geschieht z.B. in sog. Garnreinigern in Umspulmaschinen. Diese sind mit Messköpfen ausgestattet, die die Messung von einer oder mehreren Messgrössen erlauben. Ein derartiger Messkopf ist z.B. in EP 401 600 beschrieben.

Durch Verknüpfung oder andere Verarbeitung der Messgrössen wird mindestens ein Parameter $p_i$ des Garns bestimmt. Sodann wird jeder Parameter mit einem Grenzwert verglichen. Liegt einer der Parameter ausserhalb des Grenzwerts, so wird ein Fehlerzustand angenommen. Die Verarbeitung des Garns wird abgebrochen und es wird z. B. ein Reinigungsschritt durchgeführt.

In CH 439 796 werden an einem Garn mehrere Parameter gemessen. Sodann werden diese Parameter logarithmiert und die Logarithmen werden addiert. Die Summe wird sodann mit einem Schwellwert verglichen. Bei diesem Verfahren findet jedoch kein Vergleich jedes Parameters mit einem ihm zugeordneten Grenzwert statt, so dass es für viele Zwecke ungeeignet ist.

In der Praxis werden an die Garnprüfung sehr hohe Anforderungen gestellt. So sollten auch noch kleine, insbesondere systematische Abweichungen von Sollwerten erkennbar sein, wie sie z.B. bei einer Garnverwechslung auftreten können.

Es stellt sich deshalb die Aufgabe, ein möglichst zuverlässiges Verfahren der eingangs genannten Art bereitzustellen.

Diese Aufgabe wird vom Verfahren gemäss Anspruch 1 gelöst.

Erfindungsgemäss wird also eine Fehlerfunktion

$$F\left(\frac{p_1 - e_1}{s_1}, \ldots, \frac{p_n - e_n}{s_n}\right) \cdot$$

berechnet. Diese ist abhängig von den Differenzen der Parameter $p_i$ von einem jeweiligen Erwartungswert $e_i$. Jede Differenz wird gewichtet mit einem Streuwert $s_i$ des jeweiligen Parameters $p_i$. Die Fehlerfunktion verknüpft die gewichteten Differenzen, und erst dann wird der Wert mit einem einzigen Grenzwert verglichen. Dies führt dazu, dass Abweichungen eines einzelnen Parameters nicht zu einem Fehlerzustand führen, sofern die übrigen Parameter nur sehr kleine Abweichungen zeigen. Im Vergleich zu konventionellen Verfahren, bei denen jeder Parameter mit einem eigenen Grenzwert verglichen wird, erreicht das erfindungsgemässe Verfahren eine höhere Zuverlässigkeit bei der Erkennung von Fehlern.

Vorzugsweise ist die Fehlerfunktion eine Summe der Form

$$F\left(\frac{p_1 - e_1}{s_1}, \ldots, \frac{p_n - e_n}{s_n}\right) = \sum_{i=1}^{n} F_i\left(\frac{p_i - e_i}{s_i}\right) \cdot$$

Mit anderen Worten wird also für jeden Parameter ein Wert $F_i$ errechnet und diese Werte werden sodann addiert. Die Art der Funktion $F_i$ hängt von der Art der Streuung des jeweiligen Parameters ab. Für einen Parameter, dessen Werte gemäss einer Normalverteilung gestreut sind, ist vorzugsweise $F_i(x) = x^2$.

Vorzugsweise wird mindestens ein Teil der Erwartungswerte $e_i$ und/oder der Streuwerte $s_i$ durch Messung des Mittelwerts bzw. der Streuung des jeweiligen Parameters $p_i$ ermittelt, so dass eine automatische Anpassung des Systems erfolgt. Es ist jedoch auch möglich, die entsprechenden Werte z.B. aufgrund von Angaben des Garnherstellers fest vorzugeben.

Aufgrund seiner hohen Empfindlichkeit gegenüber kleinen systematischen Fehlern ist das vorliegende Verfahren besonders geeignet zum Erkennen von Garnen, deren Parameter systematisch von den Parametern eines gewünschten Garns abweichen. Dies ist z.B. der Fall, wenn versehentlich ein Garn verwendet wird, welches leicht andere Eigenschaften als das gewünschte Garn besitzt.

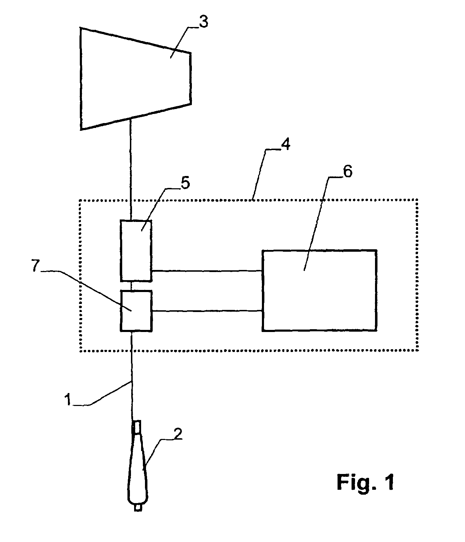

Weitere Vorteile, bevorzugte Ausführungen und Anwendungen des erfindungsgemässen Verfahrens ergeben sich aus den abhängigen Ansprüchen sowie aus der nun folgenden Beschreibung anhand von Fig. 1. Diese zeigt eine mögliche Anordnung zum Durchführen des Verfahrens.

Fig. 1 zeigt einen Garnreiniger, der an einer Umspulstation angeordnet ist. Hier wird das Garn 1 von einem oder mehreren Cops 2 auf eine Kreuzspule 3 umgespult. Dabei durchläuft es den Garnreiniger 4.

Der Garnreiniger 4 umfasst einen Messkopf 5, in welchem mehrere Parameter $p_i$ ($i$ = 1 ... $n$) des Garns gemessen werden. Mögliche Parameter sind z.B.:

- die Garndicke, ermittelt aus einer optischen Messung,
- die Haarigkeit des Garns, abgeleitet aus dem Messwert der Garndicke,
- die Farbe des Garns bzw. ein von der Garnfarbe abhängiger Wert, abgeleitet aus einer Reflektionsoder Transmissionsmessung bei mehreren Wellenlängen,
- die Masse des Garns abgeleitet aus einer kapazitiven Messung.

Die Signale des Messkopfs 5 werden einer Steuerung 6 zugeführt, wo sie analysiert werden. Die Steuerung 6 ist ausgestaltet, um einen Fehlerzustand zu erkennen. Als Fehlerzustand wird dabei z.B. eine Verdikkung des Garns in Form einer Nisse betrachtet, bei welcher der Garndurchmesser einen vorgegebenen Grenzwert übersteigt, oder eine Dünnstelle oder ein Wechsel der optischen Reflektionseigenschaften des Garns. Als Fehlerzustand soll auch erkannt werden können, wenn ein Cops 2 mit einem falschen Garn verwendet wird.

Um mindestens einen Teil der Fehlerzustände zu erkennen, berechnet die Steuerung 6 eine Fehlerfunktion F der Form

$$F\left(\frac{p_1 - e_1}{s_1}, \ldots, \frac{p_n - e_n}{s_n}\right) = \sum_{i=1}^{n}\left(\frac{p_i - e_i}{s_i}\right)^2 . \quad (1)$$

Dabei entspricht $p_i$ dem Wert des jeweiligen Parameters, $e_i$ einem Erwartungswert des Parameters und $s_i$ einem Streuwert.

Der so ermittelte Wert von F wird sodann mit einem Grenzwert G verglichen. Ist F > G, so wird ein Fehlerzustand angenommen. Der Umspulprozess wird gestoppt und es werden Schritte zum Beheben des Fehlers vorgenommen, z.B. indem ein Stück Garn herausgeschnitten wird oder indem ein Fehlersignal erzeugt wird, welches besagt, dass der Cops 2 ausgewechselt werden muss.

Gleichung (1) entspricht der Summe der Quadrate der Abweichungen der Parameter von ihrem jeweiligen Erwartungswert, gewichtet mit dem jeweiligen Streuwert. Sind die Werte der Parameter $p_i$ voneinander unabhängig und normalverteilt, so lässt sich zeigen, dass der Wert von F eng verknüpft ist mit der statistischen Wahrscheinlichkeit, dass die gemessenen Parameter $p_1$ bis $p_n$ einer rein zufälligen statistischen Schwankung entsprechen.

Je nach Verteilung der Parameter $p_i$ kann $F$ auch eine andere Form haben, z.B.

$$F\left(\frac{p_1 - e_1}{s_1}, \ldots, \frac{p_n - e_n}{s_n}\right) = \sum_{i=1}^{n} F_i\left(\frac{p_i - e_i}{s_i}\right) . \quad (2)$$

Die Funktion $F_i$ kann anstelle einer Quadratfunktion z.B. auch die Bestimmung des Absolutwerts darstellen, d.h.

$$F_i\left(\frac{p_i - e_i}{s_i}\right) = \left|\frac{p_i - e_i}{s_i}\right| , \quad (3)$$

falls starke Abweichungen des entsprechenden Parameter $p_i$ statistisch wahrscheinlich sind und nicht so stark gewichtet werden sollen.

Die Fehlerfunktion kann auch nicht direkt der Summe gemäss Gleichung (2) entsprechen, sondern lediglich die Berechnung der Summe und sodann weitere Schritte umfassen, z.B. indem der Summenwert auf der rechten Seite der Gleichung (2) noch einer linearen oder nichtlinearen Transformation unterworfen wird.

Zur Ermittlung der Parameter $p_i$ werden Messgrössen $m_i$ bestimmt. Beispielsweise kann die Garndicke aus einer Messgrösse bestimmt werden, die von der Breite des Schattens abhängt, den das beleuchtete Garn auf einen Detektor wirft. Um höhere Genauigkeit zu erhalten, werden die Parameter vorzugsweise durch Mittelung der jeweiligen Messgrösse über eine gegebene Garnlänge $L$ bestimmt.

Die Länge $L$ beträgt vorzugsweise mindestens 10 Meter, so dass lokale Streuungen unterdrückt werden. Die Obergrenze der Länge $L$ ist gegeben durch die Garnlänge, welche bei einem Fehler maximal zurückgespult wird. In der Regel beträgt der entsprechende Wert maximal ca. 100 Meter.

Wird eine Mittelung über so grosse Garnlängen $L$ durchgeführt, so wird die Vorrichtung nach Fig. 1 nebst der Bedingung $F > G$ zusätzlich noch weitere Kriterien verwenden, um kurze Fehlerzustände zu erkennen. So können zum Erkennen von Nissen oder anderen lokalen Fehlerzuständendie momentanen Messwerte $m_i$ oder eine Verknüpfung mehrerer Messwerte mit einem geeigneten Toleranzwert verglichen werden. Dies erlaubt es einerseits, mit der Bedingung $F > G$ lang anhaltende Fehler zu erkennen, wobei in diesem Falle eine Garnverwechslung vermutet wird und der jeweilige Cops 2 auszuwechseln ist. Andererseits können über den Vergleich des bzw. der Messwerte mit dem Toleranzwert lokale Fehler erkannt werden, die dann z.B. in einer Schneide- und Spleissvorrichtung 7 des Garnreinigers entfernt werden können.

Die Erwartungswerte $e_i$ und/oder die Streuwerte $s_i$ können vorgegeben werden, z.B. aufgrund von Werten, die der Garnhersteller angibt. Sie können jedoch von der Vorrichtung gemäss Fig. 1 auch selbst bestimmt werden.

Zum Ermitteln des Erwartungswerts $e_i$ kann der jeweilige Parameter $p_i$ über eine Länge $M$ des Fadens gemittelt werden, wobei die Länge $M$ mehr als 100 Meter betragen sollte. Die Mittelung kann auch gewichtet werden, z. B. derart, dass die neuesten Werte von pi einen stärkeren Einfluss auf den Mittelwert haben als ältere Werte.

Zum Ermitteln des Streuwerts $s_i$ kann die Streuung des jeweiligen Parameters $p_i$ über eine Länge $M$ des Fadens ermittelt werden. Auch hier beträgt die Länge $M$ möglichst mehr als 100 Meter.

Durch das hier beschriebene Verfahren wird es, wie erwähnt, möglich, auch geringe Abweichungen der Parameter noch zu erkennen, so dass z.B. zuverlässig festgestellt werden kann, ob aufgrund einer Garnverwechslung versehentlich Garn mit leicht von den Sollwerten abweichenden Eigenschaften verwendet wird.

**Patentansprüche**

1. Verfahren zum Prüfen der Qualität eines Garns, bei welchem mehrere Parameter $p_1$ bis $p_n$ des Garns gemessen und verwendet werden, um einen Fehlerzustand zu erkennen, **dadurch gekennzeichnet, dass** eine Fehlerfunktion

$$F\left(\frac{p_1 - e_1}{s_1}, \ldots, \frac{p_n - e_n}{s_n}\right)$$

berechnet wird, welche abhängig ist von den Differenzen der Parameter $p_i$ von einem jeweiligen Erwartungswert $e_i$ gewichtet mit einem Streuwert $s_i$, und dass zum Ermitteln des Fehlerzustands der Wert der Fehlerfunktion mit einem Grenzwert verglichen wird.

2. Verfahren nach Anspruch 1, **dadurch gekennzeichnet, dass** die Fehlerfunktion eine Summe der Form

$$F\left(\frac{p_1 - e_1}{s_1}, \ldots, \frac{p_n - e_n}{s_n}\right) = \sum_{i=1}^{n} F_i\left(\frac{p_i - e_i}{s_i}\right)$$

ist, oder dass die Fehlerfunktion die Berechnung einer derartigen Summe umfasst.

3. Verfahren nach Anspruch 2, **dadurch gekennzeichnet, dass** gilt

$$F\left(\frac{p_1 - e_1}{s_1}, \ldots, \frac{p_n - e_n}{s_n}\right) = \sum_{i=1}^{n} \left(\frac{p_i - e_i}{s_i}\right)^2$$

4.  Verfahren nach einem der vorangehenden Ansprüche, **dadurch gekennzeichnet, dass** mindestens ein Teil der Erwartungswerte $e_i$ ermittelt wird durch Mitteln des jeweiligen Parameters $p_i$, und insbesondere durch Mitteln über eine Garnlänge von mindestens 100 Metern.

5.  Verfahren nach einem der vorangehenden Ansprüche, **dadurch gekennzeichnet, dass** mindestens ein Teil der Streuwerte $s_i$ abgeleitet werden aus einer gemessenen Streuung des jeweiligen Parameter $p_i$.

6.  Verfahren nach einem der vorangehenden Ansprüche, **dadurch gekennzeichnet, dass** mindestens ein Teil, vorzugsweise alle, der Parameter $p_i$ gemessen wird durch Mittelung einer Messgrösse $m_i$ über eine gegebene Garnlänge.

7.  Verfahren nach Anspruch 6, **dadurch gekennzeichnet, dass** die gegebene Garnlänge mindestens 10 Meter und vorzugsweise höchstens 100 Meter beträgt.

8.  Verfahren nach einem der Ansprüche 6 oder 7, **dadurch gekennzeichnet, dass**, zusätzlich zum Vergleich der Fehlerfunktion mit dem Grenzwert, zur Ermittlung von kurzen Fehlerzuständen mindestens eine Messgrösse mi oder eine Verknüpfung von Messgrössen mit einem Toleranzwert verglichen wird, wobei, wenn die Fehlerfunktion den Grenzwert übersteigt, auf eine Garnverwechslung geschlossen wird, und, wenn die Messgrösse oder die Verknüpfung von Messgrössen den Toleranzwert übersteigt, auf eine lokale Fehlstelle geschlossen und diese entfernt wird.

9.  Verfahren nach einem der vorangehenden Ansprüche, **dadurch gekennzeichnet, dass** bei einem Fehlerzustand das Garn gestoppt wird.

10. Verfahren nach einem der vorangehenden Ansprüche, **dadurch gekennzeichnet, dass** mindestens einer der Parameter eine Dicke des Garns ist.

11. Verwendung des Verfahrens nach einem der vorangehenden Ansprüche zum Erkennen von Garnen, deren Parameter systematisch von den Parametern eines gewünschten Garns abweichen, und insbesondere zum Anzeigen einer Garnverwechslung.

**Claims**

1.  Method for the verification of the quality of a yarn in connection with which several parameters $p_1$ to $p_n$ of the yarn are measured and used to detect an error state, **characterised in that** an error function

$$F\left(\frac{p_1 - e_1}{s_1}, \ldots, \frac{p_n - e_n}{s_n}\right)$$

is calculated which depends on the differences of the parameters $p_i$ from a particular expected value $e_i$ weighted with a distribution coefficient $s_i$ and that for the determination of the error state the value of the error function is compared with a limiting value.

2.  Method according to claim 1, **characterised in that** the error function is a sum of the form

$$F\left(\frac{p_1 - e_1}{s_1}, \ldots, \frac{p_n - e_n}{s_n}\right) = \sum_{i=1}^{n} F_i\left(\frac{p_i - e_i}{s_i}\right)$$

or that the error function comprises the calculation of such a sum.

3. Method according to claim 2, **characterised in that**

$$F\left(\frac{p_1 - e_1}{s_1}, \ldots, \frac{p_n - e_n}{s_n}\right) = \sum_{i=1}^{n}\left(\frac{p_i - e_i}{s_i}\right)^2$$

applies.

4. Method according to one of the preceding claims, **characterised in that** at least a part of the expected values $e_i$ is determined by means of taking the average of the respective parameter $p_i$ and in particular by means of taking the average over a yarn length of at least 100 meters.

5. Method according to one of the preceding claims, **characterised in that** at least a part of the distribution coefficients $s_i$ is derived from a measured distribution of the respective parameter $p_i$.

6. Method according to one of the preceding claims, **characterised in that** at least a part of, preferably all of the parameters $p_i$ are measured by means of taking the average of a measured variable $m_i$ over a given yarn length.

7. Method according to claim 6, **characterised in that** the given yarn length is at least 10 meters and preferably at most 100 meters.

8. Method according to one of the claims 6 or 7, **characterised in that**, in addition to the comparison of the error function to the limiting value, for the determination of short error states, at least one measured variable $m_i$ or a combination of measured variables is compared to a tolerance value, wherein, if the error function exceeds the tolerance value, a yarn mix-up is concluded and if the measured variable or the combination of measured variables exceeds the tolerance value, a local defect is concluded and the same is removed.

9. Method according to one of the preceding claims, **characterised in that** the yarn is stopped in case of an error state.

10. Method according to one of the preceding claims, **characterised in that** at least one of the parameters is a thickness of the yarn.

11. Use of the method according to one of the preceding claims for the detection of yarns the parameters of which deviate systematically from the parameters of a desired yarn and in particular for reporting a yarn mix-up.

**Revendications**

1. Procédé pour contrôler la qualité d'un fil textile, dans lequel plusieurs paramètres $P_1$ à $P_n$ du fil sont mesurés et utilisés pour reconnaître un état d'erreurs, **caractérisé en ce qu'**une fonction d'erreurs

$$F\left(\frac{P_1 - e_1}{S_1}, \ldots, \frac{P_n - e_n}{S_n}\right)$$

est calculée, laquelle dépend des différences des paramètres $P_i$ d'une valeur escomptée $e_i$ concernée, pondérée par une valeur de dispersion $S_i$, et **en ce que** la valeur de la fonction d'erreurs est comparée avec une valeur limite pour déterminer l'état d'erreurs.

2. Procédé selon la revendication 1, **caractérisé en ce que** la fonction d'erreurs est une somme de la forme

$$F\left(\frac{P_1 - e_1}{S_1}, ..., \frac{P_n - e_n}{S_n}\right) = \sum_{i=1}^{n} F_1\left(\frac{P_i - e_i}{S_i}\right)$$

ou **en ce que** la fonction d'erreurs comprend le calcul d'une telle somme.

3. Procédé selon la revendication 2, caractérisé en que s'applique

$$F\left(\frac{P_1 - e_1}{S_1}, ..., \frac{P_n - e_n}{S_n}\right) = \sum_{i=1}^{n} \left(\frac{P_i - e_i}{S_i}\right)^2$$

4. Procédé selon une des revendications précédentes, **caractérisé en ce qu'**au moins une partie des valeurs escomptées $e_i$ est déterminée par des moyennes du paramètre $P_i$ concerné, et en particulier par des moyennes sur une longueur de fil d'au moins 100 mètres.

5. Procédé selon une des revendications précédentes, **caractérisé en ce qu'**au moins une partie des valeurs de dispersion $S_i$ sont dérivées d'une dispersion mesurée du paramètre $P_i$ concerné.

6. Procédé selon une des revendications précédentes, **caractérisé en ce qu'**au moins une partie, de préférence la totalité, des paramètres $P_i$, est mesurée en faisant la moyenne d'une grandeur mesurée $m_i$ sur une longueur de fil donnée.

7. Procédé selon la revendication 6, **caractérisée en ce que** la longueur de fil donnée est au moins de 10 mètres et de préférence au plus de 100 mètres.

8. Procédé selon une des revendications 6 ou 7, **caractérisé en ce que**, outre la comparaison de la fonction d'erreurs avec la valeur limite, au moins une grandeur mesurée $m_i$ ou une combinaison de grandeurs mesurées est comparée avec une valeur de tolérance pour déterminer de brefs états d'erreurs, dans lequel quand la fonction d'erreurs dépasse la valeur limite, on conclut à une confusion de fils, et quand la grandeur mesurée ou la combinaison de grandeurs mesurées dépasse la valeur de tolérance, on conclut à un défaut local et celui-ci est éliminé.

9. Procédé selon une des revendications précédentes, **caractérisé en ce qu'**en cas d'état d'erreurs, le fil est arrêté.

10. Procédé selon une des revendications précédentes, **caractérisé en ce qu'**au moins un des paramètres est l'épaisseur du fil.

11. Utilisation du procédé selon une des revendications précédentes pour la reconnaissance de fils dont les paramètres s'écartent systématiquement des paramètres d'un fil souhaité, et en particulier pour l'indication d'une confusion de fils.

**Fig. 1**